(19) Europäisches Patentamt
European Patent Office
Office européen des brevets

(11) Veröffentlichungsnummer: **0 091 593 A1**

# (12) EUROPÄISCHE PATENTANMELDUNG

(21) Anmeldenummer: 83102979.8

(22) Anmeldetag: 25.03.83

(51) Int. Cl.³: C 07 D 475/04
C 07 D 475/08, C 07 D 475/00
A 01 N 47/36

(30) Priorität: 10.04.82 DE 3213507
21.05.82 DE 3219145
28.07.82 DE 3228100

(43) Veröffentlichungstag der Anmeldung: 19.10.83 Patentblatt 83/42

(84) Benannte Vertragsstaaten: AT BE CH DE FR GB IT LI NL SE

(71) Anmelder: CELAMERCK GmbH & Co. KG
Binger Strasse 173
D-6507 Ingelheim am Rhein(DE)

(72) Erfinder: Mengel, Rudolf, Dr. Dipl.-Chem.
Schützenpfad 22
D-6507 Ingelheim(DE)

(72) Erfinder: Schröder, Ludwig, Dr. Dipl.-Chem.
Frankenstrasse 7
D-6507 Ingelheim(DE)

(72) Erfinder: Stransky, Werner, Dr. Dipl.-Chem.
Im Hippel 24
D-6535 Gau-Algesheim(DE)

(72) Erfinder: Linden, Gerbert, Dr. Dipl.-Landw.
Turnierstrasse 44
D-6507 Ingelheim(DE)

(72) Erfinder: Schneider, Gerhart, Dipl.-Biol.
Schleifmühlenweg 7a
D-6109 Mühltal 1(DE)

(72) Erfinder: Lust, Sigmund, Dr.
Klappacher Strasse 2f
D-6100 Darmstadt(DE)

## (54) N-Pteridinylharnstoffe, ihre Herstellung und Verwendung.

(57) Es werden neue N-Pteridinylharnstoffe der Formel

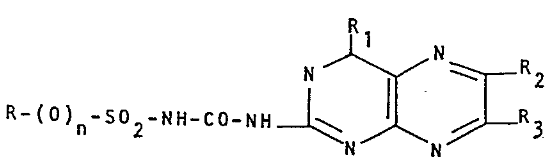

(I)

angegeben (die Substituenten sind in der Beschreibung definiert), ihre Herstellung nach konventionellen Verfahren und ihre Verwendung zur Bekämpfung unerwünschten Pflanzenwuchses.

EP 0 091 593 A1

Croydon Printing Company Ltd.

Die Erfindung betrifft neue N-Pteridinylharnstoffe, ihre Herstellung nach an sich bekannten Methoden sowie die Verwendung der neuen Verbindungen bei der Bekämpfung unerwünschten Pflanzenwuchses.

Es wurde gefunden, daß die neuen N-Pteridinylharnstoffe der Formel

$R-(O)_n-SO_2-NH-CO-NH$ 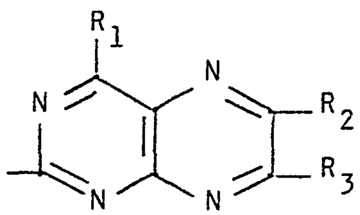 (I)

und ihre Salze mit Basen überraschenderweise selektiv gegen zahlreiche Unkräuter und Ungräser stark wirksam sind.

In der obigen Formel und im folgenden steht

n für die Zahlen 0 oder 1,

R (a) für den Rest

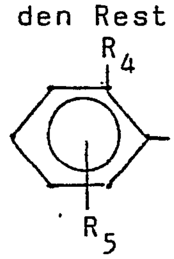 oder 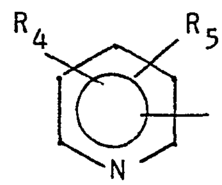

(II) (III)

oder

(b) für einen geradkettigen oder verzweigten $C_1$-$C_{12}$-Alkylrest, der auch durch Sauerstoff unterbrochen oder ein- oder mehrfach halogensubstituiert sein kann, oder einen ungesättigten Kohlenwasserstoffrest mit bis zu 12 C-Atomen und mit bis zu drei Doppelbindungen; für einen gegebenenfalls ungesättigten und/oder halogensubstituierten oder niederalkylsubstituierten cycloaliphatischen Rest mit 3 bis 7 Ringgliedern und insgesamt bis zu 12 C-Atomen; oder für den Rest $CH_2Q$, worin Q CN, 2,2-Dichlorcyclopropyl, $COOR_6$ oder auch einen gegebenenfalls ein- oder mehrfach gegebenenfalls gemischt fluor-, chlor-, brom-, niederalkyl- niederalkoxy-, niederalkylthio-, $COOR_6$- oder cyanosubstituierten Phenylrest bedeutet;

$R_1$ für Wasserstoff, Niederalkyl, Niederalkoxy, Niederalkylthio, Halogen, Amino, Mononiederalkylamino, Di-niederalkylamino;

$R_2$ und $R_3$, die gleich oder verschieden sein können, für Wasserstoff oder Niederalkyl;

$R_4$ für Halogen, Niederalkoxycarbonyl, gegebenenfalls halogensubstituiertes Niederalkyl und Niederalkoxy, Niederalkylthio, Cyano, gegebenenfalls niederalkylsubstituiertes $C_3$-$C_7$-Cycloalkyl, Nitro, Di(niederalkyl)amino, $XSO_2R_7$, CO-NH-($C_1$-$C_4$)Alkyl,CON($C_1$-$C_4$-Alkyl)$_2$, -O-Allyl;

$R_5$ für Wasserstoff, Halogen, Niederalkoxycarbonyl, Niederalkyl, Niederalkoxy, Niederalkylthio, Trifluormethyl, Cyano, gegebenenfalls niederalkylsubstituiertes $C_3C_7$-Cycloalkyl, Nitro, Di(niederalkyl)amino;

$R_6$ für Niederalkyl;

$R_7$ für Niederalkyl, gegebenenfalls niederalkoxy- oder ein- bis dreifach halogensubstituiert;

X für Sauerstoff, NH oder N(Niederalkyl) oder eine direkte C-S-Bindung.

Die Niederalkyl-, Niederalkoxy- und Niederalkylthiogruppen in den obigen Definitionen können geradkettig oder verzweigt sein. Sie enthalten vorzugsweise 1 bis 4 Kohlenstoffatome, d.h. Methyl, Ethyl, n-Propyl, i-Propyl und die verschiedenen Butyle.
Hervorzuheben sind die entsprechenden $C_1$-$C_2$-Gruppen.
Die längerkettigen aliphatischen Reste enthalten bevorzugt bis zu 6 C-Atome, und können verzweigt sein.
Falls R die Gruppe der Formel III bedeutet, steht n vor allem für die Zahl 0.

In den Pteridylgruppen stehen $R_2$ und $R_3$ vor allem für Wasserstoff oder Methyl, $R_1$ steht vor allem für Wasserstoff, Methyl, Methoxy, Chlor, Methylamino, Amino oder Dimethylamino.

Unter "Halogen" werden Fluor, Chlor, Brom und Jod verstanden, bevorzugt Fluor, Chlor und Brom, insbesondere Chlor.

Die neuen Verbindungen können nach folgenden, an sich bekannten Verfahren hergestellt werden:

1. Man setzt ein Sulfonylisocyanat der Formel

$$R-(O)_n-SO_2 - NCO \qquad (IV)$$

worin n und R die obige Bedeutung haben, mit einem Amin der Formel

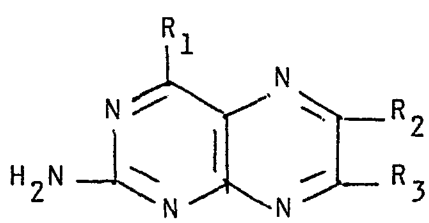

(V)

um, worin $R_1$, $R_2$ und $R_3$ die obige Bedeutung haben.

Die Umsetzung erfolgt zweckmäßig in einem inerten aprotischen Lösungsmittel wie Methylenchlorid, Acetonitril, wobei das Isocyanat vorgelegt und unter Rühren, gegebenenfalls unter Kühlung, die Aminkomponente nach und nach zugegeben wird. Die Umsetzung wird bei Raumtemperatur oder auch bei erhöhter Temperatur (bis zur Siedetemperatur des Reaktionsgemischs) zuende geführt, das Reaktionsprodukt isoliert und gewünschtenfalls nach üblichen Verfahren gereinigt. Es ist zweckmäßig, möglichst wasserfrei zu arbeiten.

Die als Ausgangsstoffe benötigten Sulfonylisocyanate der Formel IV können nach üblichen Verfahren erhalten werden, z.B. entsprechend J. Org. Chem. 39, S. 1597 ff, (1974).

Die als Ausgangsstoffe benötigten Phenoxysulfonylisocyanate der Formel IV werden zweckmäßig nach dem von Lohaus, Chem. Ber. 105, 2791 - 2799 (1972), angegebenen Verfahren hergestellt. Im allgemeinen brauchen diese Verbindungen vor der Weiterverarbeitung nicht gereinigt zu werden.

Die Phenyl- bzw. Pyridylsulfonylisocyanate der Formel IV sind bekannt oder können analog den bekannten Verbindungen dieses Typs hergestellt werden, z.B. nach DE-PS 817 602, US-PS 3 379 758, E-PS 21 641 oder H. Ulrich, Chem. Ber. 65, 369 (1965).

Die als Ausgangsstoffe benötigten Pteridine der Formel V können nach an sich bekannten Verfahren erhalten werden (W. Pfleiderer et al, Chem. Ber. 93, 2015 (1960) und 103, 722 (1970)).

2. Man setzt ein Carbamat der Formel

$$R-(O)_n-SO_2-NH-CO-OC_6H_5 \qquad (VI),$$

worin n und R die obige Bedeutung haben, mit einem Amin der Formel V um.

Die Carbamate der Formel VI können aus den Amiden der Formel

$$R-(O)_n-SO_2-NH_2 \qquad (VII)$$

und Diphenylcarbonat in Gegenwart einer Base in an sich bekannter Weise erhalten werden. Auch die Verbindungen der Formel VII sind nach üblichen Methoden herstellbar.

Gewünschtenfalls werden aus den nach 1. oder 2. erhaltenen Verbindungen der Formel I mit geeigneten Alkali- oder Erdalkaliverbindungen oder mit organischen Basen die entsprechenden Salze hergestellt. Dies geschieht vorzugsweise durch Umsetzung mit der berechneten Menge der Base und Isolierung des entstehenden Salzes.

Die Verbindungen der Formel I sind herbizid wirksam. Sie können vor und nach dem Auflaufen gegen zahlreiche Unkräuter und Ungräser eingesetzt werden, z.B. gegen Ackersenf, Klettenlabkraut, Kamille, Ackerfuchsschwanz, Hühnerhirse, Flughafer, Amaranth, Erdmandelgras, Nußgras.

Die gute Selektivität der neuen Verbindungen ermöglicht es, die Unkraut- bzw. Ungrasbekämpfung in zahlreichen Kulturen durchzuführen, beispielsweise in Weizen, Mais, Reis, Gerste, Kartoffeln, Tomaten, Sonnenblumen, Erbsen, Bohnen, Rüben, Baumwolle.

Für die Anwendung werden die Verbindungen der Formel I in an sich bekannter Weise mit üblichen Hilfs- und oder Trägerstoffen zu gebräuchlichen Formulierungen verarbeitet, z.B. zu Emulsionskonzentraten oder Suspensionspulvern, bei denen der Wirkstoffgehalt zwischen 10 und 95 Gewichtsprozent liegt und die für die Ausbringung mit Wasser bis zur gewünschten Wirkstoffkonzentration verdünnt werden. Jedoch können auch unverdünnt anwendbare Präparate hergestellt werden, etwa Granulate und Stäube. Hier liegt der Wirkstoffgehalt zwischen 0,01 und 10 Gewichtsprozent, vorzugsweise zwischen 0,1 und 3 Gewichtsprozent.

Formulierungsbeispiele

(Angabe der Zusammensetzung in Gewichtsprozent)

1. Stäubemittel

0,3 % einer Verbindung der Formel I
1,0 % Methylcellulose
98,7 % Talkum

2. Suspensionspulver

25 % einer Verbindung der Formel I
55 % Kaolin
10 % kolloidale Kieselsäure
9 % Calciumligninsulfonat
1 % Natriumtetrapropylenbenzolsulfonat

3. Suspensionspulver

95 % einer Verbindung der Formel I
4 % Calciumligninsulfonat
1 % Natriumtetrapropylenbenzolsulfonat

4. Emulsionskonzentrat

10 % einer Verbindung der Formel I
80 % Dimethylformamid
6,5 % Tensiofix AS (Emulgator)
3,5 % Tensiofix DS (Emulgator)

Aus den Konzentraten 2., 3., 4. werden durch Vermischen mit Wasser Spritzbrühen hergestellt, die im allgemeinen zwischen 0,001, vorzugsweise 0,01 und 0,5 % Wirkstoff enthalten.

Die Herstellung der Verbindungen der Formel I sowie der Ausgangsstoffe wird im folgenden näher erläutert:

Beispiel 1

N-(4-Methoxypteridin-2-yl)-N'-(2-chlorphenylsulfonyl)-harnstoff

Zu einer Lösung von 1,77 g (0,01 mol) 2-Chlorphenylsulfonylisocyanat in 20 ml Acetonitril werden 2 Tropfen Triethylamin und 1,5 g (0,0069) 2-Amino-4-methoxypteridin zugefügt. Die Mischung wird 30 Minuten unter Rückfluß erhitzt, abgekühlt und mit Diisopropylether versetzt. Nach zehnminütigem Stehen wird der Niederschlag abgesaugt.
Ausbeute: 2,25 g (87 % d.Th.); Fp. 212°C.

Beispiel 2

N-(4-Dimethylamino-6,7-dimethylpteridin-2-yl)-N'-(2-carbomethoxyphenylsulfonyl)-harnstoff

Eine Mischung auf 1 g 2-Amino-4-dimethylamino-6,7-dimethylpteridin und 1,3 g 2-Carbomethoxyphenylsulfonylisocyanat wird in 10 ml Acetonitril 30 Minuten bei Raumtemperatur gerührt, dann 3 bis 4 Minuten auf Siedetemperatur erhitzt. Nach dem Abkühlen wird die Mischung mit Diisopropylether digeriert und abfiltriert.
Ausbeute: 1,7 g (81 %d.Th.);
Fp. 196°C.

Beispiel 3

N-(4-Methoxy-6,7-dimethylpteridin-2-yl)-N'-(2-carbomethoxyphenylsulfonyl)-harnstoff

1,02 g (0,005 mol) 2-Amino-4-methoxy-6,7-dimethylpteridin und 1,92 g (0,008 mol) 2-Carbomethoxyphenylsulfonylisocyanat werden in 20 ml Acetonitril nach Zugabe von 2 Tropfen Triethylamin 45 Minuten unter Rückfluß erhitzt, nach dem Abkühlen mit Isopropylether versetzt. Nach 30 Minuten wird abgesaugt. Die schwer lösliche Substanz kann in Dimethylformamid gelöst und mit Ethanol ausgefällt werden.
Ausbeute: 1,95 g (85 % d.Th.);
Fp. 181°C.

Beispiel 4

N-(4-Methoxypteridin-2-yl)-N'-(2-fluorphenoxysulfonyl)-harnstoff

0,88 g (0,005 mol) 2-Amino-4-methoxypteridin werden in 40 ml Methylenchlorid suspendiert und 1,2 g (0,0055 mol) 2-Fluorphenoxysulfonylisocyanat schnell zugegeben. Es bildet sich eine klare Lösung, die kurz auf Siedetemperatur erwärmt, 10 Minuten nachgerührt und dann eingeengt wird.
Der Rückstand wird mit Diisopropylether und wenig Aceton verrieben, abgesaugt und getrocknet.
Ausbeute 1,95 g, hellgelbe Kristalle,
Fp. 135°C.

Beispiel 5

N-(4-Methoxypteridin-2-yl)-N'-(2-trifluormethylphenylsulfonyl)-harnstoff

0,88 g (0,005 mol) 2-Amino-4-methoxypteridin werden in 40 ml Methylenchlorid mit 1,30 g 2-Trifluormethylphenylsulfonylisocyanat (0,0052 mol) entsprechend Beispiel 4 umgesetzt.
Ausbeute 2,0 g hellgelbe Kristalle (94 % d.Th.);
Fp. 187°C.

Beispiel 6

N-(4-Dimethylaminopteridin-2-yl)-N'-(2-chlorphenyl - sulfonyl)-harnstoff

1,3 g (0,0068 mol) 2-Amino-4-dimethylaminopteridin in 40 ml Acetonitril werden mit 1,5 g (0,0070 mol) 2-Chlorphenylsulfonylisocyanat versetzt und unter Rühren und Rückfluß 4 Stunden erhitzt. Man saugt ab, engt das Filtrat ein und verreibt mit wenig Aceton. Insgesamt erhält man 2,05 g (74 %d.Th.) der Titelverbindung;
hellgelbe Kristalle,
Fp. 232°C.

Beispiel 7

N-(4-Dimethylaminopteridin-2-yl)-N'-(2-methoxycarbonylphenylsulfonyl)-harnstoff

1,3 g (0,0068 mol) 2-Amino-4-dimethylaminopteridin in 40 ml Acetonitril werden mit 1,7 g 2-Methoxycarbonylphenylsulfonylisocyanat 4 Stunden unter Rückfluß erhitzt. Man saugt heiß ab und erhält 2,5 g bräunliche Kristalle,
Fp. 219°C;
Ausbeute 85 % d.Th.

Beispiel 8

N-(4-Methoxypteridin-2-yl)-N'-(2-carbomethoxyphenylsulfonyl)-harnstoff

1,68 g (0,005 mol) N-(2-Carbomethoxyphenylsulfonyl)-phenylcarbamat (herstellbar aus dem entsprechenden Sulfonamid und Diphenylcarbonat) und 0,85 g (0,0048 mol) 2-Amino-4-methoxypteridin werden in 30 ml abs. Dioxan 8 Stunden unter Rückfluß gekocht. Nach dem Einengen wird der Rückstand zweimal mit Natriumhydrogencarbonatlösung und mehrmals mit Wasser digeriert. Man gibt Wasser und wenig Aceton zu und bringt durch Reiben zur Kristallisation. Es werden 1,85 g hellbraune Kristalle erhalten, die aus Ethanol/DMF umkristallisiert werden können.
Ausbeute 80 % d.Th.;
Fp. 188°C.

Beispiel 9

N-(4-Methoxypteridin-2-yl)-N'-(2,2,2-trichlorethoxy-sulfonyl)-harnstoff

Zu einer Suspension von 0,88 g (0,005 mol) 2-Amino-4-methoxypteridin in 40 ml abs. Methylenchlorid werden unter Rühren 1,35 g (0,0053 mol) 2,2,2-Trichlorethoxysulfonylisocyanat zugegeben. Nach ca. 10 Minuten hat sich eine klare Lösung gebildet. Sie wird auf Siedetemperatur erwärmt und nach 10 Minuten eingeengt. Der Rückstand wird mit Diisopropylether und wenig Aceton verrieben, abgesaugt und getrocknet; gelbe Kristalle.
Ausbeute: 2,05 g (95 % d.Th.);
Fp. ab 93°C (Zers.).

Entsprechend den Beispielen können auch die Verbindungen der Formel I erhalten werden, die in den folgenden Tabellen aufgeführt sind:

Tabelle I

Verbindungen der Formel

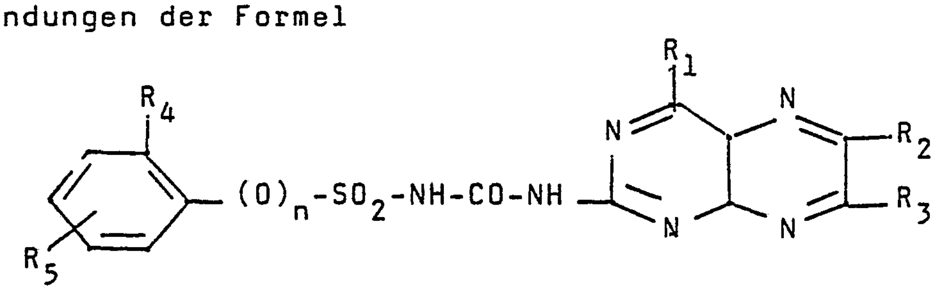

| Nr. | n | $R_4$ | $R_5$ | $R_1$ | $R_2$ | $R_3$ | Fp °C |
|---|---|---|---|---|---|---|---|
| 1 | 0 | $CF_3$ | H | $OCH_3$ | H | H | 187 |
| 2 | 0 | $OCHF_2$ | H | $OCH_3$ | H | H | |
| 3 | 0 | F | 4-F | $CH_3$ | H | H | |
| 4 | 0 | Cl | 4-$CH_3$ | F | H | H | |
| 5 | 0 | $CH_3$ | 4-Cl | Cl | $CH_3$ | $CH_3$ | |
| 6 | 0 | $OC_2H_5$ | H | Cl | H | H | |
| 7 | 0 | $COOC_2H_5$ | H | $OCH_3$ | H | H | |
| 8 | 0 | Br | H | $NH_2$ | H | H | |
| 9 | 0 | Cl | 4-Cl | $NHCH_3$ | H | H | |
| 10 | 0 | $OCHF_2$ | H | $N(CH_3)_2$ | H | H | |
| 11 | 1 | $CH_3$ | H | $OCH_3$ | $CH_3$ | $CH_3$ | |
| 12 | 1 | Cl | H | $NHCH_3$ | H | H | |
| 13 | 1 | $OC_2H_5$ | H | Cl | H | H | |
| 14 | 1 | $COOCH_3$ | H | $OCH_3$ | H | H | 188 |

| Nr. | n | $R_4$ | $R_5$ | $R_1$ | $R_2$ | $R_3$ | Fp °C |
|---|---|---|---|---|---|---|---|
| 15 | 1 | $OCHF_2$ | H | $N(CH_3)_2$ | H | H | |
| 16 | 1 | Cl | H | Br | H | H | |
| 17 | 1 | Br | H | $CH_3$ | H | H | |
| 18 | 1 | $OCF_3$ | H | $OCH_3$ | H | H | |
| 19 | 1 | $SCH_3$ | $4-CH_3$ | $OCH_3$ | H | H | |
| 20 | 1 | F | $5-CH_3$ | $NHCH_3$ | H | H | |
| 21 | 0 | $CF_3$ | H | $N(CH_3)_2$ | H | H | 192 |
| 22 | 1 | $COOCH_3$ | H | $N(CH_3)_2$ | H | H | 153 |
| 23 | 1 | F | H | $OCH_3$ | H | H | 135 |
| 24 | 1 | $COOCH_3$ | H | $OCH_3$ | H | H | 153 |
| 25 | 0 | $COOCH_3$ | H | $N(CH_3)_2$ | H | H | 215 |
| 26 | 0 | $CF_3$ | H | $N(CH_3)_2$ | H | H | 187 |
| 27 | 0 | $CF_3$ | H | $OCH_3$ | H | H | 214 |
| 28 | 0 | $COOCH_3$ | H | $OCH_3$ | H | H | 188 |
| 29 | 0 | $CF_3$ | H | $NH_2$ | H | H | 275 |
| 30 | 0 | $COOCH_3$ | H | $NH_2$ | H | H | 225 |
| 31 | 0 | CL | H | $NH_2$ | H | H | 270 |
| 32 | 0 | Cl | H | $N(C_2H_5)_2$ | H | H | 221 |
| 33 | 0 | $OCH_3$ | 5-Br | $N(CH_3)_2$ | H | H | 248 |
| 34 | 0 | Cl | H | $NH_2$ | $CH_3$ | $CH_3$ | 232 |
| 35 | 0 | Cl | H | $N(CH_3)_2$ | $H/CH_3$* | | 204 |
| 36 | 0 | $COOCH_3$ | H | $N(CH_3)_2$ | $H/CH_3$* | | 215 |
| 37 | 0 | $CON(CH_3)_2$ | H | $N(CH_3)_2$ | H | H | 193 |

*Produkt ist ein Gemisch, bei dem sich z.T. in 6-und z.T. in 7-Position eine Methylgruppe befindet.

Tabelle II

Verbindungen der Formel

$CCl_3-CH_2-O-SO_2-NH-CO-NH-$ 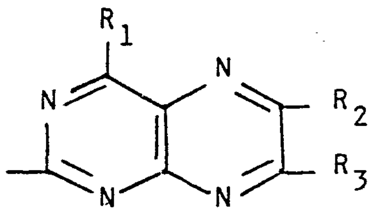

| Nr. | $R_1$ | $R_2$ | $R_3$ |
|---|---|---|---|
| 1 | $OCH_3$ | H | H |
| 2 | $OCH_3$ | H | H |
| 3 | $CH_3$ | H | H |
| 4 | F | H | H |
| 5 | Cl | $CH_3$ | $CH_3$ |
| 6 | Cl | H | H |
| 7 | $OCH_3$ | H | H |
| 8 | $NH_2$ | H | H |
| 9 | NHCH | H | H |
| 10 | $N(CH_3)_2$ | H | H |
| 11 | $OCH_3$ | $CH_3$ | $CH_3$ |
| 12 | $NHCH_3$ | H | H |
| 13 | CL | H | H |
| 14 | $OCH_3$ | H | H |
| 15 | $N(CH_3)_2$ | H | H |
| 16 | Br | H | H |
| 17 | $CH_3$ | H | H |
| 18 | $OCH_3$ | H | H |
| 19 | $OCH_3$ | H | H |
| 20 | $NHCH_3$ | H | H |
| 21 | $N(CH_3)_2$ | H | H |
| 22 | $N(CH_3)_2$ | H | H |

Tabelle III

Verbindungen der Formel

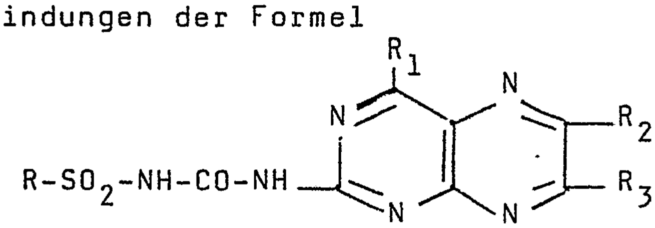

| Nr. | $R_1$ | $R_2$ | $R_3$ | R | Fp °C |
|---|---|---|---|---|---|
| 1 | $OCH_3$ | H | H | $CH_3$ | |
| 2 | $OCH_3$ | H | H | 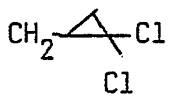 | |
| 3 | $CH_3$ | H | H | $n\text{-}C_4H_9$ | |
| 4 | F | H | H | $t\text{-}C_4H_9$ | |
| 5 | Cl | $CH_3$ | $CH_3$ | $n\text{-}C_3H_7$ | |
| 6 | Cl | H | H | $n\text{-}C_4H_9$ | |
| 7 | $OCH_3$ | H | H | $i\text{-}C_3H_7$ | |
| 8 | $NH_2$ | H | H | $n\text{-}C_6H_{13}$ | |
| 9 | $NHCH_3$ | H | H | $n\text{-}C_3H_7$ | |
| 10 | $N(CH_3)_2$ | H | H | $n\text{-}C_{12}H_{25}$ | |
| 11 | $OCH_3$ | $CH_3$ | $CH_3$ | $C_2H_5$ | |
| 12 | $NHCH_3$ | H | H | 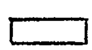 | |
| 13 | Cl | H | H | 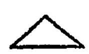 | |
| 14 | $OCH_3$ | H | H | $CH_2{=}CH\text{-}CH_2$ | |
| 15 | $N(CH_3)_2$ | H | H | $CH_2{=}CH\text{-}CH_2$ | |
| 16 | Br | H | H | 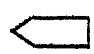 | |
| 17 | $CH_3$ | H | H | 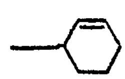 | |
| 18 | $OCH_3$ | H | H | $i\text{-}C_3H_7$ | |

| Nr. | $R_1$ | $R_2$ | $R_3$ | R | Fp °C |
|---|---|---|---|---|---|
| 19 | $OCH_3$ | H | H | $CH_3-CH=CH-CH_2$ | |
| 20 | $NHCH_3$ | H | H | $n-C_6H_{13}$ | |
| 21 | $N(CH_3)_2$ | H | H | $n-C_3H_7$ | |
| 22 | $N(CH_3)_2$ | H | H | $i-C_4H_9$ | |

Tabelle IV

Verbindungen der Formel I

| Nr. | n | R | $R_1$ | $R_2$ | $R_3$ | Fp °C |
|---|---|---|---|---|---|---|
| 1 | 1 | $CCl_3-CH_2$ | $OCH_3$ | H | H | 92 (Zers.) |
| 2 | 0 | 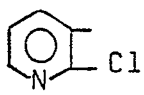 | $N(CH_3)_2$ | H | H | 207 |
| 3 | 0 | 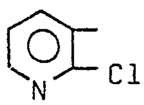 | $OCH_3$ | H | H | 227 |
| 4 | 0 | 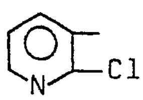 | $NH_2$ | H | H | 230 |
| 5 | 0 | $n-C_3H_7$ | $N(CH_3)_2$ | H | H | 178 |

Patentansprüche

1. Verbindungen der Formel

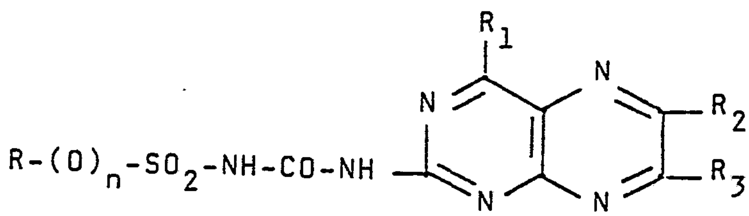

(I)

in der

n für die Zahlen 0 oder 1,

R (a) für den Rest

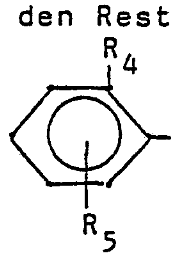

oder

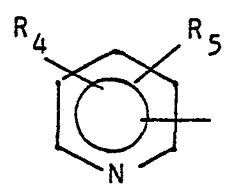

(II) (III)

oder

(b) für einen geradkettigen oder verzweigten $C_1$-$C_{12}$-Alkylrest, der auch durch Sauerstoff unterbrochen oder ein- oder mehrfach halogensubstituiert sein kann, oder einen ungesättigten Kohlenwasserstoffrest mit bis zu 12 C-Atomen und mit bis zu drei Doppelbindungen; für einen gegebenenfalls ungesättigten und/oder halogensubstituierten oder niederalkylsubstituierten cycloaliphatischen Rest mit 3 bis 7 Ringgliedern und insgesamt bis zu 12 C-Atomen; oder für den Rest $CH_2Q$, worin Q CN, 2,2-Dichlorcyclopropyl, $COOR_6$ oder auch einen gegebenenfalls ein- oder mehrfach gegebenenfalls gemischt fluor-, chlor-, brom-, niederalkyl- niederalkoxy-, niederalkylthio-, $COOR_6$- oder cyanosubstituierten Phenylrest bedeutet;

$R_1$ für Wasserstoff, Niederalkyl, Niederalkoxy, Niederalkylthio, Halogen, Amino, Mono-niederalkylamino, Di-niederalkylamino;

$R_2$ und $R_3$, die gleich oder verschieden sein können, für Wasserstoff oder Niederalkyl;

$R_4$ für Halogen, Niederalkoxycarbonyl, gegebenenfalls halogensubstituiertes Niederalkyl und Niederalkoxy, Niederalkylthio, Cyano, gegebenenfalls niederalkylsubstituiertes $C_3$-$C_7$-Cycloalkyl, Nitro, Di(niederalkyl)amino, $XSO_2R_7$, CO-NH-($C_1$-$C_4$)Alkyl, CON($C_1$-$C_4$-Alkyl)$_2$, -O-Allyl;

$R_5$ für Wasserstoff, Halogen, Niederalkoxycarbonyl, Niederalkyl, Niederalkoxy, Niederalkylthio, Trifluormethyl, Cyano, gegebenenfalls niederalkylsubstituiertes $C_3$-$C_7$-Cycloalkyl, Nitro, Di(niederalkyl)amino;

$R_6$ für Niederalkyl;

$R_7$ für Niederalkyl, gegebenenfalls niederalkoxy- oder ein- bis dreifach halogensubstituiert;

X für Sauerstoff, NH oder N(Niederalkyl) oder eine direkte C-S-Bindung..

und ihre Salze.

2. Verbindungen nach Anspruch 1, in denen R den Rest der Formel II oder III bedeutet.

3. Verbindungen nach Anspruch 1, in denen R die unter (b) genannte Bedeutung hat.

4. Verbindungen nach Anspruch 1 oder 3, in denen R die Gruppe $CCl_3CH_2$ bedeutet und n für 1 steht.

5. Verbindungen nach Anspruch 1 bis 4, in denen $R_1$ Wasserstoff, Chlor, Methyl, Methoxy, Methylamino, Amino,Dimethylamino, $R_2$ und $R_3$ Wasserstoff oder Methyl sind.

6. Verbindungen nach Anspruch 1 bis 5, in denen die niederen Alkyl-, Alkoxy- und Alkylthiogruppen 1 oder 2 C-Atome und die längeren aliphatischen Kohlenwasserstoffreste bis zu 6 C-Atome umfassen.

7. Verbindungen nach Anspruch 1, 3, 5 oder 6, worin R für (2,2-Dichlorcyclopropyl)methyl steht.

8. Herbizide Mittel, gekennzeichnet durch einen Gehalt an einer Verbindung nach Anspruch 1 bis 7 neben üblichen Hilfs- und/oder Trägerstoffen.

9. Verwendung von Verbindungen nach Anspruch 1 bis 7 bei der Bekämpfung unerwünschten Pflanzenwachstums.

10. Verfahren zur Herstellung von Verbindungen nach Anspruch 1 bis 7, dadurch gekennzeichnet, daß man

a) ein Isocyanat der Formel

$$R-(O)_n-SO_2-NCO \qquad (IV)$$

worin n und R die obige Bedeutung haben, mit einem Amin der Formel

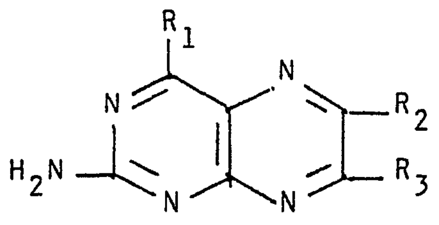

(V)

umsetzt, worin $R_1$, $R_2$ und $R_3$ die obige Bedeutung haben, oder daß man

b) ein Carbamat der Formel

$$R-(O)_n-SO_2-NH-CO-OC_6H_5 \qquad (VI)$$

worin R und n die obige Bedeutung haben, mit einem Amin der Formel V umsetzt

und gewünschtenfalls die nach a) und b) erhaltenen Verbindungen der Formel I in üblicher Weise in Salze überführt.

0091593

Europäisches Patentamt

# EUROPÄISCHER RECHERCHENBERICHT

Nummer der Anmeldung

EP 83 10 2979

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 015 683 (DU PONT DE NEMOURS)<br>* Insgesamt *<br>--- | 1-10 | C 07 D 475/04<br>C 07 D 475/08<br>C 07 D 475/00<br>A 01 N 47/36 |
| A | EP-A-0 023 140 (DU PONT DE NEMOURS)<br>* Insgesamt *<br>--- | 1-10 | |
| A | EP-A-0 004 163 (DU PONT DE NEMOURS)<br>* Insgesamt *<br>--- | 1-10 | |
| A,D | CHEMISCHE BERICHTE, Band 103, 1970, Seiten 722-734, Weinheim, DE.<br>G. KONRAD et al.: "Synthese und Eigenschaften von 2.4-Diamino-pteridinen und ihren 5.6.7.8-Tetrahydro-Derivaten"<br>----- | | **RECHERCHIERTE SACHGEBIETE (Int. Cl. 3)**<br>C 07 D 475/00<br>A 01 N 47/00 |

Der vorliegende Recherchenbericht wurde für alle Patentansprüche erstellt.

| Recherchenort | Abschlußdatum der Recherche | Prüfer |
|---|---|---|
| DEN HAAG | 08-07-1983 | NUYTS A.M.K.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82